(19) Europäisches Patentamt
European Patent Office
Office européen des brevets

(11) **EP 1 201 245 B1**

(12) **EUROPEAN PATENT SPECIFICATION**

(45) Date of publication and mention
of the grant of the patent:
**21.11.2007 Bulletin 2007/47**

(51) Int Cl.:
*A61K 38/17* (2006.01)  *A61K 38/16* (2006.01)
*A61K 38/00* (2006.01)  *A23L 1/304* (2006.01)
*A23L 1/308* (2006.01)

(21) Application number: **00906682.0**

(22) Date of filing: **03.03.2000**

(86) International application number:
**PCT/JP2000/001291**

(87) International publication number:
**WO 2000/051627 (08.09.2000 Gazette 2000/36)**

(54) **USE OF FUNCTIONAL ORAL PREPARATIONS**

VERWENDUNG FUNKTIONELLER ORALER PRÄPARATE

UTILISATION DES PREPARATIONS ORALES FONCTIONNELLES

(84) Designated Contracting States:
**DE FR GB**

(30) Priority: **04.03.1999 JP 5699299**
**04.03.1999 JP 5699399**

(43) Date of publication of application:
**02.05.2002 Bulletin 2002/18**

(73) Proprietor: **SEIREN CO., LTD.**
**Fukui-shi,**
**Fukui 918-8003 (JP)**

(72) Inventors:
• **Sasaki, Masahiro**
**Fukui-shi,**
**Fukui 918-8003 (JP)**
• **Yamada, Hideyuki**
**Fukui-shi,**
**Fukui 918-8003 (JP)**
• **Nomura, Masakazu**
**Fukui-shi,**
**Fukui 918-8003 (JP)**

(74) Representative: **Hamer, Christopher K. et al**
**Mathys & Squire**
**120 Holborn**
**London EC1N 2SQ (GB)**

(56) References cited:
EP-A- 0 841 065          JP-A- 1 256 351
JP-A- 2 177 864

• PATENT ABSTRACTS OF JAPAN vol. 1997, no. 02, 28 February 1997 (1997-02-28) & JP 8 268905 A (NAGASHIMA TAKAYUKI), 15 October 1996 (1996-10-15)
• SADAHIRO SASAKI: 'Dimethylhidrazine yuhatsu mouse daichou gan hasshou ni oyobosu kinu tanpaku shitsu sericin no eikyou' JOURNAL OF JAPAN SOCIETY FOR BIOSCIENCE, BIOTECHNOLOGY AND AGROCHEMISTRY (JSBA) vol. 73, no. EXTRA ISSUE, 05 March 1999, page 140, 2P240D, XP002946271
• KAYASHITA JUN. ET AL: 'Buckwheat protein extract suppression of the growth depression in rats induced by feeding amaranth(food red no. 2)' BIOSCIENCE BIOTECHNOLOGY AND BIOCHEMISTRY vol. 60, no. 9, 1996, pages 1530 - 1531, XP002928051
• KAYASHITA JUN. ET AL.: 'Feeding of buckwheat protein extract reduces hepatic triglyceride concentration, adipose tissue weight and hepatic lipogenesis in rat' JOURNAL OF NUTRITIONAL BIOCHEMISTRY vol. 7, 1996, pages 555 - 559, XP002928052
• S. KIRIYAMA ET AL.: 'Shin eiyou kagaku' KABUSHIKI KAISHA ASAKURA SHOTEN 01 March 1998, pages 95 - 106, XP002946272
• DATABASE WPI Week 199818, Derwent Publications Ltd., London, GB; Class B04, AN 1998-196391 & JP 10 029 909 A (SHINKO HYOHAKU KOGYO KK) 03 February 1998

EP 1 201 245 B1

## Description

### [Background of the Invention]

The present invention is broadly concerned with use of functional oral preparations and intended particularly for useful application as low-digestible food/drink additives, health supplements and/or colon cancer preventives.

### [Prior Art]

The westernized eating habits of the Japanese people have resulted in a yearly decrease in their intake of dietary fiber, a food component that cannot be digested by human digestive enzymes. On the other hand, however, dietary fiber has been reviewed for its beneficial effects on health such as its low calorie diet, blood sugar level improvement and constipation prevention/treatment. Further, recently increased concern among people in Japan for their health and beauty care has led to patent proposal and marketing of foods and drinks containing various types of dietary fiber.

For example, Published Japanese Patent Application JP-A-7-313120 has proposed a drink containing dietary fiber composed of a low molecular weight version of gluco-mannan (taken from the tuber of Japanese vegetable "konnyaku").

Many other types of dietary fiber proposed in Japanese patents for use in such foods and drinks include an extract from a certain type of mushroom (JP-A-199849), low molecular weight pectin (JP-A-6-169724) and alginates (JP-A-2-303468).

A high percentage of dietary fiber currently available for commercial use in foods and drinks is water-soluble. Dietary fiber of this type has a propensity to form a gel in water, which is so viscous that it requires reduction of its viscosity for addition to a food or drink, making the manufacture of the food or drink complicated. In addition, water-soluble dietary fiber presents problems such as causing a large amount of gas to be generated in the abdominal region when ingested.

Furthermore, dietary fiber, which functions to adsorb excess sugar, cholesterol and other substances in the body, causing them to be excreted outside it. This fact suggests a possibility of the fiber's even excreting vitamins and minerals necessary for the body at the same time. The prevention of such a possibility is extremely important, especially not only because the Japanese people's intake of calcium has been said to be insufficient, but also because sufficient intake of minerals have been recently promoted to avoid risks of associated diseases. Notwithstanding this, however, easy and careless intake of any particular minerals alone in large quantities cannot be said to be an efficient method of mineral supply to the body, as well as may involve a danger of causing new nutrition-related problems such as inhibited absorption of other minerals into the body. Therefore, it is an important matter to discuss the dietary fiber's action of accelerating the intestinal absorption of minerals from the standpoint of their intake from food well thought for its nutritious balance.

Meanwhile, statistics of death causes in Japan show that cancer is the leading cause of death in this country, while indicating that colon cancer accounts for a high percentage of all the deaths from cancer. This trend in cancer deaths in this nation is expected to become increasingly strong, causing colon cancer to place higher than stomach cancer in the list of cancer-caused deaths in the 21st century.

An increased incidence of colon cancer in Japan is considered greatly associated with by environmental factors. Among such factors is the diet of its people rapidly changed to a western style one based on animal fats and proteins, which, for their digestion and absorption in the body, involve the secretion of large quantities of bile acids alleged to form carcinogens.

In the meantime, free radical and activated oxygen have recently attracted attention as carcinogenic agents, which are known to occur in the body under the influence of heat, light, cigarette smoke, exhaust gas, UV and other environmental factors, or physiologically. These agents thus formed in the body are assumed to attack the lipids, proteins and nucleic acids constituting the biomembranes and tissues, causing them to undergo oxidation reaction with their resultantly accumulated damage leading to the development of cancer.

Colon cancer is said to allow its complete treatment to be achieved if it is detected at its early stage. Notwithstanding this, however, colon cancer is so difficult to detect at its polypous stage prior to its progression to its cancerous stage that as countermeasures against its development, various attempts have been made to prevent intake of the above-mentioned carcinogenic agents into the body or to achieve their quick discharge out of it.

Among the subjects of discussion as part of such attempts with particular attention is dietary fiber. Referring to "food components resistant to digestion by human digestive enzymes" on a collective basis, dietary fiber, when ingested in the body as food, is assumed to increase the amount of feces evacuated from the bowels and accelerate the peristaltic movement of the intestinal tract, thereby shortening the time for transit of intestinal contents for consequent reduction in the amount of putrescent and carcinogenic matter formed in the intestine and in the time for their contact with the intestinal membrane to control the development of cancer. Dietary fiber is generally classified into two types - water-insoluble and water-soluble. The former type of dietary fiber includes cellulose, lignin, hemicellulose A, hemicellulose C, chitin and collagen, while the latter type of dietary fiber comprises pectin, glucomannan (such as represented by konnyaku mannan), sodium alginate, carrageenan, agar-agar, carboxymethylcellulose, low-digestible dextrin and polydextrose. Although dietary fiber is said to have such health benefits as mentioned above, however, it varies largely in its effects depending on its type, whether water-soluble or insoluble. In addition, besides being a non-nutrient food component, dietary fiber has many problems to be addressed, such as its excessive intake resulting in a sensation of fullness in the abdominal region or a laxative evacuation of the bowels.

Among other substances than dietary fiber that have been reported useful colon cancer preventives are antioxidants such as polyphenol, which, however, require intake in such a high dosage extremely impossible to attain in everyday life that many colon cancer preventives now available on the market have used dietary fiber as their active ingredients.

JP-A-1256351 describes the use of silk protein, for example fibroin and sericin, in the preparation of drinks which are intended to be "extremely soft and pleasant to the palate", and additionally provide a "smooth texture".

JP-A-2177864 relates to the prevention of hangover or protection from alcoholic hepatopathy using hydrolyzed silk protein.

EP 0841065A describes use of sericin to exert an antioxidising effect and an inhibiting action on tyrosinase activity. Such use is in the context of improving the longevity of foods, for example seafood, fruits and vegetables; and to the promotion of cosmetic bleaching, and other associated disorders such as freckles, pimples, eczema and the like.

Finally, JP-A-10029909 discloses use of sericin, dissolved in an ionic water, as being generally useful for cosmetics, drugs, food or fibre reforming agents.

[Objects of the Invention]

Accordingly, it is an object of the present invention to provide and use functional oral preparations improved by solving the above-mentioned problems involved in the prior art.

It is another object of the present invention to provide and use novel low-digestible food/drink additives and health supplements which not only are effective in body weight control and constipation prevention/treatment, but also have the action of accelerating the intestinal absorption of minerals such as calcium, iron, magnesium and zinc ingested from food.

An further object of the present invention includes the provision and use of novel colon cancer preventives which not only are safe and highly effective in preventing colon cancer at small dosage levels, but also advantageous in its easy and convenient intake through daily diet.

[Summary of the Invention]

The inventors of the present invention have discovered that sericin and its hydrolyzed product are resistant to digestion by digestive enzymes in the body and are effective in body weight control and constipation prevention/treatment, while having the characteristic effect of accelerating the intestinal absorption of calcium and other minerals ingested, and that they, when ingested orally as such or through food or drink, have excellent effects of controlling the body weight, preventing/treating constipation and accelerating the intestinal absorption of calcium and other minerals.

In addition, the inventors of the present invention have also discovered that sericin and its hydrolyzed product are effective in preventing colon cancer at low dosage levels and have successfully obtained highly safe and effective colon cancer preventives with sericin and/or its hydrolyzed product as their active ingredients.

Therefore, the present invention resides in use of a functional oral preparation comprising sericin and/or its hydrolyzed product, in the manufacture of a medicament for the prevention or inhibition of colon cancer, body weight control, prevention or inhibition of constipation and/or accelerating intestinal mineral absorption, which may be used in form of food/drink additives and health supplement.

[Embodiments of the Invention]

The health supplements as used herein means embodiments of the present invention, which, when ingested not through food or drink with them added to it, but by oral intake as they are directly or in such states as supported on carriers, can demonstrate their effectiveness in body weight control, constipation prevention/treatment and/or acceleration of the intestinal absorption of minerals.

Sericin generally used in the present invention is preferably prepared from silkworm cocoons or raw silk. The preparation of sericin as its non-hydrolyzed product for use according to the present invention can be extracted from silkworm cocoons or raw silk by generally practiced extracting methods, such as the one described below, which allows extraction of sericin as a single protein from silkworm cocoons or raw silk with a purity of 90% or higher.

Specifically, sericin contained in silkworm cocoons or raw silk is extracted with water and recovered by either of the following two methods (1) and (2), for example:

(1) Mixing the extract with water-soluble organic solvent such as methanol, ethanol or dioxane for precipitation of sericin, which is then subjected to filtration, followed by drying to obtain it in powder form.

(2) Processing the extract with ultrafiltration or reverse osmosis membrane for separation of sericin as proposed in Japanese Patent JP-A-4-202435, followed by drying to obtain it in powder form.

On the other hand, the preparation of sericin as its hydrolyzed product for use according to the present invention can be extracted from silkworm cocoons or raw silk by generally practiced extracting methods

Specifically, as an example of such methods, sericin contained in silkworm cocoons or raw silk is subjected to treatment with electrolyzed water, acid, alkali, enzyme or other appropriate substances for partial hydrolysis of sericin prior to its extraction, followed by its recovery according to either of the above-mentioned two methods, for example.

Silkworm cocoons have attracted attention for their proteins of good quality. Actually, processing of the cocoons such as their pulverization has been proposed for addition to foods as nutrients. However, sericin, which is among such cocoon proteins, has not been known to be resistant to digestion by digestive enzymes in the body and effective in body weight control and constipation prevention/treatment.

Sericin, which is hardly digestible in the body, activates the intestinal activities and functions to shorten the time for transit of feces in the intestinal tract, causing the harmful and toxic waste matter in the bowels to be quickly evacuated from the body. Sericin also has such effects as shown by dietary fiber, including controlling the intestinal absorption of excess sugars, lipids and other substances.

In addition to such direct effects on the body as mentioned above, sericin has indirect effects on the body such as its absorbed water allowing smooth defecation.

Furthermore, the inventors of the present invention have discovered that sericin has the capability of uniting with minerals, accelerating the intestinal absorption of the minerals. Sericin, hardly digestible in the stomach and other digestive tract, when thus united with minerals in the digestive tract, is assumed to reach the bowels with the minerals bound to it, then functioning to assist in their absorption into the body. The minerals used herein include, without limitation, calcium, iron, magnesium, zinc, manganese, copper, chromium, selenium, molybdenum, cobalt, nickel, arsenic, iodine, boron, bromine, fluorine, lead, lithium, silicon, vanadium, cadmium and other elements necessary for the body's health.

The sericin preparation for use according to the present invention is a naturally derived one, which is highly safe in vivo and can thus be used as additives to general foods, drinks and health food products, as well as special use foods (designated by the Japan's Ministry of Health, Labor and Welfare) such as its designated specific health foods, and other reasonably-appropriate orally ingestible foods (collectively herein referred to as "foods and drinks"), allowing its easy and convenient intake as part of daily diets. In addition, the sericin preparation for use in the present invention is water-soluble, allowing it to be easily added to foods and drinks in various forms. Further, the sericin preparation for use in the present invention can be blended with one or more of the above-mentioned minerals so that they are well-balanced in their blending before being processed as additives to foods and drinks, allowing the minerals to be efficiently ingested.

As health supplements, the sericin preparation for use in the present invention can be used in various forms, which include, without limitation, powder, solution, gel, stick, granule, capsule and tablet.

The sericin preparation for use in the present invention, which is nonpoisonous, can be daily ingested in unlimited quantities, although its daily intake of 1 to 30g is generally sufficient to achieve its such effectiveness as mentioned in the present invention.

As colon cancer preventives, the sericin preparation for use in the present invention can also be used in various forms, which include, without limitation, solution, gel, stick, granule and tablet, as well as capsule in various formulations to allow it to develop its effectiveness in the intestine efficiently. Being nonpoisonous, the sericin preparation of the present invention can be daily administered in unlimited doses as mentioned above, although its daily dosage of 1mg to 1g per kg of body weight is generally sufficient to achieve its such effectiveness as mentioned in the present invention.

[Examples of the Invention]

The present invention will now be further illustrated by the following examples, which shall not be construed to limit the scope of the present invention.

**[Preparation Example 1]**

Preparation of Sericin as its non-hydrolyzed product in powder form
1kg of silkworm cocoons was subjected to treatment in 50 liters of water at 95°C for 2 hours for extraction of sericin as its non-hydrolyzed product from the cocoons. The resultant extract was filtered through a filter with an average pore diameter of 0.2μm for removal of the aggregate in the extract. The resultant filtrate was treated with reverse osmosis membrane for its demineralization to obtain a colorless transparent aqueous solution of sericin as its non-hydrolyzed product at a concentration of 0.2%. The aqueous solution was then condensed with an evaporator until its sericin concentration became approximately 2% before being freeze-dried to obtain 100g of non-hydrolyzed sericin in powder form with an average molecular weight of 100,000 and a purity of 95% or higher (hereinafter referred to as "Sericin H").

**[Preparation Example 2]**

Preparation of sericin as its hydrolyzed product in powder form
1kg of silkworm cocoons was subjected to treatment in 50 liters of a 0.2% solution of sodium carbonate in water (adjusted to pH 11-12) at 95°C for 2 hours for extraction of sericin as its hydrolyzed product from the cocoons. The resultant extract was filtered through a filter with an average pore diameter of 0.2μm for removal of the aggregate in the extract. The resultant filtrate was treated with reverse osmosis membrane for its demineralization to obtain a colorless transparent aqueous solution of sericin as its hydrolyzed product at a concentration of 0.2%. The aqueous solution was then condensed with an evaporator until its sericin concentration became approximately 2% before being freeze-dried to obtain 100g of hydrolyzed sericin in powder form with an average molecular weight of 20,000 and a purity of 90% or higher (hereinafter referred to as "Sericin L").

**[Test Example 1]** Test of sericin for its digestibility

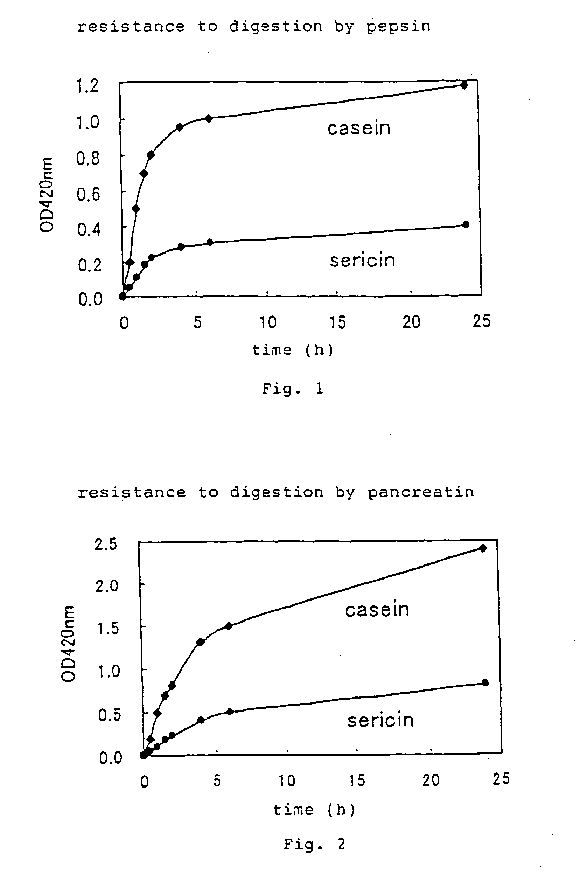

As a specimen for the digestibility test, Sericin L or casein was used. The specimen was adjusted using hydrochloric acid and diluted with distilled water to 100ml so that it became a 0.5% solution with a pH of 2.0. 5mg of pepsin (Sigma, St. Louis, MO, USA), a digestive enzyme produced in the stomach, was added to the solution, which was then incubated at 37°C for 24 hours, during which it was sampled at adequate intervals. After the incubation was completed, the 100ml specimen solution was adjusted using sodium hydrogen carbonate to 0.1% (pH8.5). Then, 2mg of pancreatin (Sigma, St. Louis, MO, USA), an enzyme produced in the duodenum, was added to the 0.1% (pH8.5) solution, which was then incubated at 37°C for 24 hours, during which it was sampled at adequate intervals.
The samples thus taken from the solution during its incubation were measured for their peptide concentrations using the TNBS (2,4,6-trinitrobenzensulfonic acid) method; 0.1ml of 30% TCA (trichloroacetic acid) was added to 0.5ml of each sample, which was then subjected to centrifugation for separation of the solution. To 100 μl of the resultant supernatant, 3ml of a 7% sodium hydrogen carbonate solution and 2ml of a 0.1% TNBS solution were added. The solution was then warmed at 37°C for 2 hours before being measured by spectrophotometer for absorbance at 420nm.
The results of the measurements are shown in Fig.1 and Fig.2, both of which represent changes in the absorbance of the specimen with its absorbance prior to its incubation in both cases assumed to be "0" (zero). The test was intended to confirm *in vitro* the resistance of sericin to digestion by pepsin and pancreatin, both of which are digestive enzymes produced in the body. The TNBS method is based on the reaction of a TNBS reagent with the amino terminal group of protein, meaning that the less resistant the specimen is to digestion by the enzymes (or the higher its digestibility), the larger the quantity of peptides it forms, resulting in its increased reactivity with the TNBS reagent. Fig.1 and Fig.2 have confirmed that compared to casein, sericin is highly resistant to digestion by either of pepsin or pancreatin.
[Test Example 2] Test of sericin for its effect on intestinal transit As experimental animals for the test, 5-week old male ICR mice (Charles River Japan, Yokohama, Japan) were used, which were divided into groups of 10. The experimental animals were housed in metal cages in a room at a controlled temperature of 24 ± 1°C under an environment with a 12 hour light-dark cycle (lights on from 8:00 to 20:00/lights off from 20:00 to 8:00 the next day). For one week as their preliminary feeding period, the mice were fed a commercially available solid diet (MF, Oriental Yeast, Tokyo, Japan). On the day before the start of the test, the animals were fasted overnight before being fed for one hour, from which those almost equal in their food intakes during the one hour period were sorted out into groups of 8 for use in the test. The animals were orally administered BTB as a marker for their intestinal transit performance (at a dose of 0.35ml of its 0.3% solution per animal) together with Sericin L (at a dose of 2g per kg body weight) or casein as a control (at a dose of 2g per kg body weight). Feces were collected from the animals at adequate intervals during 25 hours after the administration of the marker. During this 25-hour period, the mice were fed for one hour each at 6 hours and 23 hours after the marker administration. Both during their preliminary feeding and experiment periods, the mice were given ad libitum access to distilled water. The feces collected were measured for fecal BTB levels before being dried for 24 hours under reduced pressure for measurement of their dry weights. The measurements were evaluated for significant difference using Student's t-test.

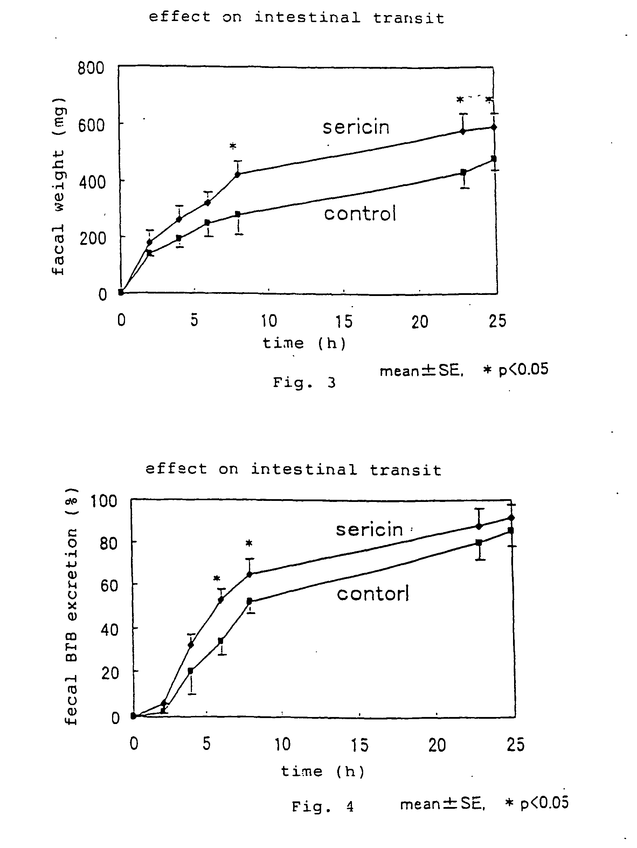

The results showed that the fecal weight in the sericin group was significantly larger than that in the control group (Fig.3). The comparison of the feces of the control and sericin groups in their physical characters indicated that compared to the former, the latter was somewhat large and soft, although not suggesting any symptoms of diarrhea such as watery or mushy stool.

In the control group, BTB was first excreted in the feces at 2 to 4 hours after the administration with its subsequent fecal excretion recorded at 33.5% of its total administered dose within 6 hours, 53.5% within 8 hours and 88.3% within 25 hours. On the other hand, the sericin group showed a significant increase in the fecal BTB excretion at 4 to 8 hours after the administration compared to that in the control group, confirming that sericin has the effect of decreasing the intestinal transit time (Fig.4).

**[Test Example 3]** Test of sericin for its effect on constipation improvement

As experimental animals for the test, 3-week old male Sprague-Dawley rats (Charles River Japan, Yokohama, Japan) were used, which were divided into groups of 13. The experimental animals were housed in metal cages in a room at a controlled temperature of $24\pm1$°C under an environment with a 12 hour light-dark cycle. For 2 weeks as their preliminary feeding period, the rats were fed a commercially available solid diet (MF, Oriental Yeast, Tokyo, Japan) being divided into two groups - sericin and control, which were fed their respective experimental diets (specified in Table 1) for 7 days. Each group consisted of 14 rats, all of which were daily fed during a limited time of 14:00 to 17:00. Both during their preliminary feeding and experiment periods, the rats were given ad libitum access to distilled water. At 17: 00 on the 7th day after the start of the test, each group was further divided into two groups so that the rats of the two groups were equal in their average body weights before being administered atropine chloride (at a dose of 0.5mg per kg body weight) or physiological saline as a control by intraperitoneal injection. Feces were collected from the rats 6 hours after the injection (at 17:00 to 23:00) and dried under reduced pressure for measurement of their dry weights. The measurements were evaluated for significant difference using Duncan's multiple comparison test.

Many cases of human constipation are what can be categorized as functional constipation, which is caused by decreased release of acetylcholine from the parasympathetic nerve terminal to the intestinal smooth muscle, resulting in inhibited digestive tract movement and anal function. Atropine used in the test, which is an alkaloid derived from belladonna of the nightshade family, is known to block parasympathetic functions and competitively antagonize the activities of acetylcholine and acetylcholine-like drugs, consequently inducing functional constipation.

The results of the test in Table 2 show that sericin contributes to improvement in the digestive tract function deteriorated as a result of the atropine administration, proving to be effective in improving constipation.

Table 1

Composition of experimental diets (per 100g)

|  | Control group | Sericin group |
| --- | --- | --- |
| Casein | 25.0 | 20.0 |
| Sericin L | -- | 5.0 |
| L-cystine | 0.1 | 0.1 |
| Corn oil | 10.0 | 10.0 |
| Mineral mix | 3.5 | 3.5 |
| Vitamin mix | 1.0 | 1.0 |
| Choline tartrate | 0.2 | 0.2 |
| Cellulose | 5.0 | 5.0 |
| Saccharose | 20.0 | 20.0 |
| Corn starch | 35.2 | 35.2 |

Table 2

|  | Physiological saline | | Atropine | |
| --- | --- | --- | --- | --- |
|  | Control group | Sericin group | Control group | Sericin group |
| Food intake (g/ld) | $15.0\pm0.6$ | $16.1\pm0.8$ | $15.0\pm0.6$ | $16.1\pm0.8$ |
| Fecal weight (g) | $0.59\pm0.06$[ab] | $0.78\pm0.11$[a] | $0.32\pm0.08$[b] | $0.71\pm0.12$[a] |

(continued)

| | Physiological saline | | Atropine | |
|---|---|---|---|---|
| | Control group | Sericin group | Control group | Sericin group |
| Dry fecal weight (g) | $0.46 \pm 0.05^{ab}$ | $0.51 \pm 0.05^a$ | $0.21 \pm 0.06^b$ | $0.42 \pm 0.06^{ab}$ |
| Water content (%) | $23.3 \pm 2.1^b$ | $39.4 \pm 3.2^a$ | $24.5 \pm 3.4^b$ | $36.3 \pm 2.8^a$ |

Values are means$\pm$SE of measurements per group. a/b indicates significant difference (at $P<0.05$), while ab indicates no significant difference.

**[Test Example 4]** Test of sericin for its effect on fat accumulation control

As experimental animals for the test, 5-week old male ICR mice (Charles River Japan, Yokohama, Japan) were used, which were divided into groups of 12. The animals were housed in metal cages in a room at a controlled temperature of $24 \pm 1°C$ under an environment with a 12 hour light-dark cycle for 56 days, during which they were given ad libitum to the experimental diets (as shown in Table 3) and distilled water. After the end of the 8-week period, the mice were subjected to anatomy under ether anesthesia for measurement of their body weights and perirenal fat pad weights to divide the latter by the former respectively so as to determine their respective perirenal fat pad weight ratios. In addition, blood samples were collected from the animals to obtain serums from the blood samples according to the generally practiced method. The serums were measured for triglyceride concentrations using triglyceride measuring kit "Triglyceride G-Test Wako" (Wako Pure Chemical Industries, Osaka, Japan). The measurements were evaluated for significant difference using Duncan's multiple comparison test.

As can be seen from the test results as shown in Table 4, the sericin groups, compared to the control group, show increasingly controlled body weights of the mice with a rise in the sericin content of the diets fed to them, although these groups indicate no significant difference in food intake. In addition, the sericin groups showed a decrease in the perirenal fat pad weight ratios of the mice with increasing sericin concentration in the diets fed to them, which also resulted in reduction in their serum triglyceride levels, confirming that sericin has the effect of controlling fat accumulation in the body.

Table 3

Composition of experimental diets (per 100g)

| | Control group | Sericin 1% | Sericin 3% | Sericin 5% |
|---|---|---|---|---|
| Casein | 25.0 | 20.0 | 20.0 | 20.0 |
| Sericin L | -- | 1.0 | 3.0 | 5.0 |
| L-cystine | 0.1 | 0.1 | 0.1 | 0.1 |
| Corn oil | 10.0 | 10.0 | 10.0 | 10.0 |
| Mineral mix | 3.5 | 3.5 | 3.5 | 3.5 |
| Vitamin mix | 1.0 | 1.0 | 1.0 | 1.0 |
| Choline tartrate | 0.2 | 0.2 | 0.2 | 0.2 |
| Cellulose | 5.0 | 5.0 | 5.0 | 5.0 |
| Saccharose | 20.0 | 20.0 | 20.0 | 20.0 |
| Corn starch | 35.2 | 35.2 | 35.2 | 35.2 |

Table 4

| | Control group | Sericin 1% | Sericin 3% | Sericin 5% |
|---|---|---|---|---|
| Food intake (g/ld) | $4.93 \pm 0.13$ | $5.03 \pm 0.21$ | $5.11 \pm 0.17$ | $4.98 \pm 0.14$ |
| Final weight (g) | $44.3 \pm 0.6^a$ | $43.1 \pm 0.4^{ab}$ | $42.5 \pm 0.6^b$ | $41.5 \pm 0.3^b$ |
| Perirenal fat pad weight ratio (%) | $3.77 \pm 0.26^a$ | $3.67 \pm 0.27^{ab}$ | $3.21 \pm 0.31^b$ | $3.02 \pm 0.20^b$ |
| Serum triglyceride (mmol/L) | $1.01 \pm 0.03^a$ | $0.96 \pm 0.04^{ab}$ | $0.91 \pm 0.05^b$ | $0.90 \pm 0.04^b$ |

Values are means$\pm$SE of measurements per group. a/b indicates significant difference (at $P<0.05$), while ab indicates no significant difference.

**[Test Example 5]** Test of sericin for its effect on acceleration of the intestinal mineral absorption

As experimental animals for the test, 8-week old male Sprague-Dawley rats (Charles River Japan, Yokohama, Japan) were used, which were divided into groups of 8. The experimental animals were housed in metal cages in a room at a controlled temperature of $24\pm1$°C under an environment with a 12 hour light-dark cycle for 10 days, during which they were given ad libitum access to the experimental diets (as shown in Table 5) and distilled water. After the end of the 10-day period, the experimental animals were transferred to metabolism cages for measurement of their 3-day food intakes and fecal weights. In addition, the feces collected from the rats were measured for their fecal mineral concentrations using an atomic absorption spectrophotometer (Z-8000, Hitachi, Tokyo, Japan) to determine their apparent mineral absorption ratios according to the following formula:

$$\text{Mineral absorption ratio (\%)} = [(\text{Mineral intake} - \text{Fecal mineral})/\text{Mineral intake}] \times 100$$

As can be seen from the test results given in Table 6, the sericin group, compared to the control group, shows significant increase in the apparent mineral absorption ratios of the rats, confirming that sericin has the effect of accelerating the intestinal mineral absorption.

Table 5
Composition of experimental diets (per 100g)

| | Control group | Sericin group |
|---|---|---|
| Casein | 23.0 | 20.0 |
| Sericin L | -- | 3.0 |
| L-cystine | 0.1 | 0.1 |
| Corn oil | 10.0 | 10.0 |
| Mineral mix | 3.5 | 3.5 |
| Vitamin mix | 1.0 | 1.0 |
| Choline tartrate | 0.2 | 0.2 |
| Cellulose | 5.0 | 5.0 |
| Saccharose | 20.0 | 20.0 |
| Corn starch | 37.2 | 37.2 |

Table 6

| | Control group | Sericin group |
|---|---|---|
| Food intake (3days/g) | $45.5\pm1.3$ | $46.2\pm1.1$ |
| Dry fecal weight (3days/g) | $3.86\pm0.65$ | $3.95\pm0.83$ |
| Apparent mineral absorption ratio (%) | | |
| Calcium | $57.0\pm0.8$ | $65.2\pm2.5$** |
| Magnesium | $52.3\pm1.5$ | $67.3\pm0.4$** |
| Iron | $37.0\pm0.2$ | $46.2\pm1.3$** |
| Zinc | $33.5\pm1.3$ | $45.0\pm0.6$** |

Values are means $\pm$ SE of measurements per group. Double asterisk (**) indicates significant difference at $P<0.01$.

**[Test Example 6]** Test of sericin for its effect on colon cancer prevention

As experimental animals for the test, 4-week old male ICR mice (Charles River Japan, Yokohama, Japan) were used, which were divided into groups of 12. The experimental animals were housed in metal cages in a room at a controlled temperature of $24\pm1$°C under an environment with a 12 hour light-dark cycle. For one week as their preliminary feeding period, the mice were fed a commercially available solid diet (MF, Oriental Yeast, Tokyo, Japan) before they were subsequently fed with the experimental diets (as shown in Table 7) for 35 days. The animals were subcutaneously injected with 1,2-dimethylhydrazine three times at a dose of 20mg per kg body weight during the first 3 weeks after the start of the experiment. During the experiment period, the animals were given ad libitum access to the diets and distilled water. After the end of the experiment period, the animals were measured for their body weights and food intakes (as shown in Table 8).

The mice were then subjected to anatomy under ether anesthesia for blood sampling, immediately followed by removal of their large intestines. The intestines were cut open and spread out on paper towel before being formalin-fixed. The intestines were then washed with distilled water and shaken in 0.1% (w/v) methylene blue for 5 minutes before being stained for observation of their surfaces with a stereomicroscope (at X 40 magnification) to count the number of Aberrant crypt foci (ACF), precancerous lesions of the colon.

The test results are shown in Tables 8 and 9. In spite of no significant difference between the sericin and control groups in their body weights and food intakes (Table 8), the former, compared to the latter, reveals a significant decrease in the intestinal ACF count of the mice with a rise in the sericin content of the diets fed to them, confirming that sericin has the effect of inhibiting precancerous lesions of colon. (Table 9).

Table 7

|  | Control group | Sericin 1% | Sericin 3% |
| --- | --- | --- | --- |
| Casein | 23.0 | 22.0 | 20.0 |
| Sericin H | -- | 1.0 | 3.0 |
| L-cystine | 0.3 | 0.3 | 0.3 |
| Corn oil | 10.0 | 10.0 | 10.0 |
| Vitamin mix (AIN-93) | 1.0 | 1.0 | 1.0 |
| Salt mix (AIN-93) | 4.8 | 4.8 | 4.8 |
| Cellulose | 5.0 | 5.0 | 5.0 |
| Saccharose | 20.0 | 20.0 | 20.0 |
| Corn starch | 35.9 | 35.9 | 35.9 |

Table 8

|  | Food intake (g/day) | Initial weight (g) | Final weight (g) |
| --- | --- | --- | --- |
| Control | $4.4\pm0.2$ | $26.3\pm0.4$ | $36.2\pm0.6$ |
| Sericin 1% | $4.5\pm0.1$ | $25.4\pm0.3$ | $35.4\pm0.5$ |
| Sericin 3% | $4.4\pm0.3$ | $25.8\pm0.3$ | $36.5\pm0.3$ |

Values are means $\pm$ SE of measurements per group.

Table 9

|  | No. of mice | ACF count per mouse (means$\pm$SE) |
| --- | --- | --- |
| Control | 12 | $14.3\pm1.2$ |
| Sericin 1% | 12 | $8.9\pm1.1$* |
| Sericin 3% | 12 | $6.7\pm0.9$* |

Single asterisk (*) indicates significant difference at $P<0.05$.

**[Test Example 7]** Test of sericin for its effect on colon cancer prevention

This test was Test Example 6 modified with the experiment period extended to 14 weeks to induce development of intestinal cancer so as to confirm the sericin's effect of inhibiting such cancer development.

As experimental animals for the test, 4-week old male ICR mice (Charles River Japan, Yokohama, Japan) were used, which were divided into groups of 40. The experimental animals were housed in metal cages in a room at a controlled temperature of 24 $\pm$1°C under an environment with a 12 hour light-dark cycle. For one week as their preliminary feeding period, the mice were fed a commercially available solid diet (MF, Oriental Yeast, Tokyo, Japan) before they were subsequently fed with the experimental diets (as specified in Table 7 with Sericin H replaced by Sericin L) for 98 days. The animals were subcutaneously injected with 1,2-dimethylhydrazine once a week at a dose of 10mg per kg body weight during the first 10 weeks after the start of the experiment. During the experiment period, the animals were given ad libitum access to the diets and distilled water. After the end of the experiment period, the animals were measured for their body weights and food intakes (as shown in Table 10).

The mice were then subjected to anatomy under ether anesthesia for blood sampling, immediately followed by removal of their large intestines. The intestines were cut open and spread out on paper towel before being formalin-fixed. The intestines were then washed with distilled water and shaken in 0.1% (w/v) methylene blue for 5 minutes before being stained for observation of their surfaces with a stereomicroscope (at X 40 magnification) to determine the colon tumor incidence per group and the colon tumor count per mouse, both of which were evaluated for significant difference using Duncan's multiple comparison test and $\chi^{2-}$ test, respectively (as shown in Table 11).

Despite no significant difference between the sericin and control groups in their body weights and food intakes (Table 10), the former, compared to the latter, show significant decreases both in the colon tumor incidence per group and the colon tumor count per mouse (Table 11), confirming that sericin has the effect of preventing the development of colon cancer.

Table 10

|  | Food intake (g/day) | Initial weight (g) | Final weight (g) |
|---|---|---|---|
| Control | 4.3±0.1 | 26.3±0.4 | 50.2±0.6 |
| Sericin 1% | 4.5±0.1 | 25.1±0.3 | 49.3±0.5 |
| Sericin 3% | 4.4±0.1 | 25.4±0.3 | 49.7±0.5 |

Values are means±SE of measurements per group.

Table 11

|  | Colon tumor incidence per group (%) | Colon tumor count per mouse (means±SE) |
|---|---|---|
| Control | 36/40(90) | 3.52±1.0 |
| Sericin 1% | 27/40 (68)* | 1.89±0.5* |
| Sericin 3% | 20/40 (50) | 1.05±0.4* |

Single asterisk (*) indicates significant difference at P<0.05.

**[Effects of the Invention]**

The sericin preparation of the present invention, which is hardly digestible in the body, activates the functions of the stomach and intestines, while inhibits gastric and intestinal absorption of excess water, cholesterol, harmful matter, neutral fat and other substances ingested in the body, thus having significant effects on body weight control and constipation prevention/treatment. The sericin preparation according to the present invention is useful as an accelerator of the intestinal absorption of minerals such as calcium, iron, magnesium and zinc.

In addition, as a naturally derived protein, the sericin preparation of the present invention shows such high in-vivo safety that it is completely nonpoisonous even if ingested in large amounts. Furthermore, the sericin preparation of the present invention is tasteless and odorless, allowing it to be added to foods without affecting their tastes. Accordingly, the sericin preparation of the present invention enables its easy and convenient intake as part of daily diet, which, in turn, contributes to further marked development of its effectiveness.

As colon cancer preventives, the sericin preparation of the present invention shows its excellent effectiveness in inhibiting the development and growth of intestinal cancer at a low dosage.

Sericin, as the principal ingredient of the present invention, can be isolated from a solvent (water) extract of silkworm cocoons or raw silk in such an easy manner as a single protein with such a high degree of purity that its preparation according to the present invention can be obtained at a cheap cost. In addition, the sericin preparation thus obtained is greatly advantageous in that it is a colorless transparent aqueous solution, eliminating the need for its further complex processing that is otherwise required, such as its color removal.

**[Brief Description of the Drawing]**

Fig.1 is a graph showing the results of the test on the present invention for its resistance to digestion by pepsin.
Fig.2 is a graph showing the results of the test on the present invention for its resistance to digestion by pancreatin.
Fig.3 shows a graph showing the results of the test on the present invention for its effect on the intestinal transit in terms of fecal weight.
Fig.4 shows a graph showing the results of the test on the present invention for its effect on the intestinal transit in terms of fecal BTB excretion.

**Claims**

1. Use of at least one material selected from the group consisting of sericin and its hydrolysis product in the manufacture of a medicament for: the prevention or inhibition of colon cancer, body weight control, the prevention or inhibition of constipation and/or accelerating intestinal mineral-absorption.

2. Use according to Claim 1, wherein the medicament is an oral preparation.

3. Use according to Claim 2, wherein the oral preparation is in the form of a low-digestible food/drink additive.

4. Use according to Claim 2, wherein the oral preparation is in the form of a health supplement.

5. Use according to any one of Claims 1 to 4 for colon cancer prevention.

6. Use according to any one of Claims 1 to 4 for mineral absorption.

7. Use according to any one of Claims 1 to 4 and 6, wherein the medicament further contains a mineral mix.

8. Use according to any one of Claims 1 to 7, wherein sericin is naturally derived from silkworm cocoons or raw silk.

**Patentansprüche**

1. Verwendung von wenigstens einem Material, ausgewählt aus der Gruppe, bestehend aus Sericin und seinem Hydrolyseprodukt, zur Herstellung eines Medikaments zur: Vorbeugung oder Hemmung von Dickdarmkrebs, Gewichtskontrolle, Vorbeugung oder Hemmung von Obstipation und/oder Beschleunigung von intestinaler Mineralabsorption.

2. Verwendung nach Anspruch 1, wobei das Medikament eine orale Zubereitung ist.

3. Verwendung nach Anspruch 2, wobei die orale Zubereitung als schwer verdaulicher Lebensmittel/Getränke-Zusatz vorliegt.

4. Verwendung nach Anspruch 2, wobei die orale Zubereitung als Nahrungsergänzungsmittel vorliegt.

5. Verwendung nach einem der Ansprüche 1 bis 4 zur Vorbeugung von Dickdarmkrebs.

6. Verwendung nach einem der Ansprüche 1 bis 4 zur Mineralabsorption.

7. Verwendung nach einem der Ansprüche 1 bis 4 und 6, wobei das Medikament zusätzlich einen Mineralmix enthält.

8. Verwendung nach einem der Ansprüche 1 bis 7, wobei Sericin natürliches Sericin aus Seidenraupenkokons oder roher Seide ist.

**Revendications**

1. Utilisation d'au moins un matériau choisi dans le groupe constitué de séricine et de son produit d'hydrolyse dans la fabrication d'un médicament pour : la prévention ou l'inhibition d'un cancer du côlon, le contrôle du poids corporel, la prévention ou l'inhibition d'une constipation et/ou l'accélération de l'absorption intestinale de minéraux.

2. Utilisation selon la revendication 1, dans laquelle le médicament est une préparation orale.

3. Utilisation selon la revendication 2, dans laquelle la préparation orale est sous la forme d'un additif alimentaire/de boisson faiblement digestible.

4. Utilisation selon la revendication 2, dans laquelle la préparation orale est sous la forme d'un supplément bénéfique pour la santé.

5. Utilisation selon l'une quelconque des revendications 1 à 4 pour la prévention d'un cancer du colon.

6. Utilisation selon l'une quelconque des revendications 1 à 4 pour une absorption de minéraux.

7. Utilisation selon l'une quelconque des revendications 1 à 4 et 6, dans laquelle le médicament contient en outre un mélange de minéraux.

8. Utilisation selon l'une quelconque des revendications 1 à 7, dans laquelle la séricine est naturellement dérivée de cocons de vers à soie ou de soie grège.

resistance to digestion by pepsin

Fig. 1

resistance to digestion by pancreatin

Fig. 2

effect on intestinal transit

Fig. 3    mean±SE, *p<0.05

effect on intestinal transit

Fig. 4    mean±SE, *p<0.05

**EP 1 201 245 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7313120 A
- JP 199849 A
- JP 6169724 A
- JP 2303468 A
- JP 1256351 A
- JP 2177864 A
- EP 0841065 A
- JP 10029909 A
- JP 4202435 A